(19) **Europäisches Patentamt**
**European Patent Office**
**Office européen des brevets**

(11) **EP 4 483 787 A1**

(12) **EUROPEAN PATENT APPLICATION**
published in accordance with Art. 153(4) EPC

(43) Date of publication:
**01.01.2025 Bulletin 2025/01**

(21) Application number: **23760310.5**

(22) Date of filing: **14.02.2023**

(51) International Patent Classification (IPC):
**A61B 5/00** (2006.01)        **A61B 5/11** (2006.01)

(52) Cooperative Patent Classification (CPC):
**A61B 5/00; A61B 5/11**

(86) International application number:
**PCT/KR2023/002087**

(87) International publication number:
**WO 2023/163436 (31.08.2023 Gazette 2023/35)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **24.02.2022 KR 20220024454**
**10.02.2023 KR 20230017732**

(71) Applicants:
• **Plan B4U Co., Ltd.**
**Seongnam-si, Gyeonggi-do 13605 (KR)**

• **Seoul National University Hospital**
**Seoul 03080 (KR)**

(72) Inventors:
• **KIM, Ki Woong**
**Seoul 06718 (KR)**
• **LEE, Hyang Jun**
**Seoul 06356 (KR)**
• **PARK, Jeong Bin**
**Incheon 21376 (KR)**

(74) Representative: **AWA Sweden AB**
**Box 45086**
**104 30 Stockholm (SE)**

(54) **COGNITIVE IMPAIRMENT DETERMINATION METHOD AND DEVICE**

(57)     Provided is a cognitive impairment determination method that discloses a model for determining cognitive impairment based on a gait feature value measured by an accelerometer attached to a subject, discloses a configuration for determining cognitive impairment by using a model including a mathematical linear regression equation that includes a gait speed and step time variability as predictors by receiving personal data and gait data, easily and quickly screens for cognitive impairment, such as dementia, regardless of a root cause, and accurately determines even cognitive impairment in an early stage.

FIG. 11

RECEIVE PERSONAL DATA OF DETERMINATION TARGET — S201

RECEIVE AND STORE SIGNALS FROM INERTIA MEASUREMENT UNIT — S202

GENERATE GAIT DATA FROM STORED SIGNALS — S203

DETERMINE WHETHER DETERMINATION TARGET HAS COGNITIVE IMPAIRMENT BY SUBSTITUTING PERSONAL DATA AND GAIT DATA INTO COGNITIVE IMPAIRMENT DETERMINATION MODEL — S204

PROVIDE DETERMINATION RESULT TO USER — S205

EP 4 483 787 A1

**Description**

Technical Field

The present disclosure provides a cognitive impairment determination method, device, and computer program product.

Background Art

A gait of a person is related to a peripheral nervous system and a central nervous system, and as the person ages, a gait speed, a cadence, a step length, a step time, variability, and asymmetry change. The gait of the person also changes due to cognitive disorders (CD), such as mild cognitive impairment and dementia. A slow gait speed and high gait variability are associated with a prevalence rate and an incidence rate of CD. According to previous studies, a slow gait speed is associated with a brain region related to an information processing speed, whereas temporal gait variability is associated with a brain region related to cortical sensorimotor control. According to such findings, a slow gait speed is associated with risks of vascular dementia or Parkinson's dementia, whereas temporal gait variability is associated with risks of cognitive decline unrelated to cerebrovascular and/or Parkinson's disease. Motor cognitive risk syndrome, characterized by cognitive decline and a slow gait speed, is a new high-risk condition for dementia and is also reported to be associated with the risks of vascular dementia and Parkinson's dementia rather than Alzheimer's disease. Temporal gait variability is also related to cognitive decline, but there have been no previous studies that use temporal gait variability instead of a gait speed or simultaneously use a gait speed and temporal gait variability to predict or determine CD.

The absence of such studies may be due to insufficient reliable and accessible methods to measure temporal gait variability. A gait may be analyzed through various methods, but gait analysis using a laboratory-based 3-dimensional motion capture system and instrumented walkways shows low reliability because only a limited number of steps can be measured due to spatial constraints, and is expensive as a tool for screening for CD.

Accordingly, there is a need to develop an algorithm for measuring a gait by using an inexpensive wearable acceleration sensor with few spatial constraints and determining CD by using data obtained through gait measurement.

The aforementioned background technology is technical information possessed by the inventor for derivation of the present disclosure or acquired by the inventor during the derivation of the present disclosure, and is not necessarily prior art disclosed to the public before the application of the present disclosure.

Disclosure

Technical Problem

The present disclosure discloses various embodiments of cognitive impairment determination methods, devices, and computer program products. Technical problems to be achieved by present embodiments are not limited to the technical problems described above and other technical problems may be inferred from following embodiments.

Technical Solution

According to a first aspect of the present disclosure, provided is a cognitive impairment determination method in which each operation is performed by a processor, including: determining N subjects who are subject to cognitive impairment determination and obtaining personal data of the subjects; obtaining clinical outcome data for the subjects; obtaining gait data for the subjects, based on gait measurement values measured by using inertia measurement units worn on centers of body mass of the subjects; and developing a cognitive impairment determination model including a plurality of predictors, based on obtained datasets, wherein the cognitive impairment determination model includes gait speeds and step time variability included in the gait data as some of the plurality of predictors.

The obtaining of the personal data may include obtaining the personal data by receiving ages, sexes, educations, heights, weights, presence of lower limb arthritis, and mini mental state examination results of the subjects.

The obtaining of the clinical outcome data may include, based on a result of diagnosing cognitive impairment including mild cognitive impairment and dementia, according to pre-set diagnostic criteria, receiving results of classifying subjects diagnosed with cognitive impairment into a first group and classifying subjects not diagnosed with cognitive impairment into a second group.

The obtaining of the gait data may include: obtaining signals measured when the subjects walk by using the inertia measurement units worn on the centers of body mass of the subjects; and obtaining cadences, step times, gait speeds, step lengths, and step time variability, based on the obtained signals.

The developing of the cognitive impairment determination model may include: dividing the datasets into development datasets and validation datasets; developing the cognitive impairment determination model including the gait speeds and the step time variability as some of the plurality of predictors, by using binary logistic regression analysis using a portion of the gait data and a portion of the personal data as independent variables, for the development datasets; developing a comparative model including mini mental state examination scores and ages as predictors, by using binary logistic regression analysis using a portion of the personal data as independent variables, for the development datasets; and generating receiver operating characteristics (ROC) curves of the developed cognitive impairment determination model and the developed comparative model, for the development datasets and the validation datasets, and evaluating determination performance of the cognitive impairment determination model by comparing areas under the curves (AUC).

The cognitive impairment determination method may further include determining whether a determination target has cognitive impairment by using the developed cognitive impairment determination model.

The determining of whether the determination target has cognitive impairment may include: obtaining educations of the determination target; receiving a tri-axial acceleration signal and a tri-axial angular velocity signal measured by an inertia measurement unit worn on a center of body mass of the determination target and obtaining a gait speed and step time variability, based on the tri-axial acceleration signal and the tri-axial angular velocity signal; and determining whether the determination target has cognitive impairment by substituting obtained values into the cognitive impairment determination model.

According to a second aspect of the present disclosure, provided is a computer program product including one or more computer-readable recording media storing a program for performing a cognitive impairment determination method including: determining N subjects who are subject to cognitive impairment determination and obtaining personal data of the subjects; obtaining clinical outcome data for the subjects; obtaining gait data for the subjects, based on gait measurement values measured by using inertia measurement units worn on centers of body mass of the subjects; developing a cognitive impairment determination model including a plurality of predictors, based on obtained datasets; and determining whether a determination target has cognitive impairment by using the developed cognitive impairment determination model, wherein the cognitive impairment determination model includes gait speeds and step time variability included in the gait data as some of the plurality of predictors.

According to a third aspect of the present disclosure, provided is a cognitive impairment determination device including: an input and output unit configured to receive personal data and clinical outcome data of subjects and a determination target, and output determined cognitive impairment of the determination target; a network unit configured to receive gait measurement values measured by using inertia measurement units worn on centers of body mass of the subjects and the determination target; a processor configured to control the input and output unit to obtain the personal data and clinical outcome data of the subjects and the determination target, who are targets of cognitive impairment determination, obtain gait data based on the received gait measurement values, develop a cognitive impairment determination model including a plurality of predictors based on obtained datasets, and determine whether the determination target input through the developed cognitive impairment determination model has cognitive impairment; and a storage unit storing the obtained personal data, clinical outcome data, and gait data, wherein the cognitive impairment determination model includes gait speeds and step time variability included in the gait data as some of the plurality of predictors.

Other aspects, features, and advantages may become clear from the following drawings, the claims, and the detailed description of the present disclosure.

Advantageous Effects

According to the above-described technical solutions of the present disclosure, provided is a cognitive impairment determination method, in which cognitive impairment, such as dementia, is easily and quickly screened regardless of a root cause and cognitive impairment in an early stage is accurately determined, by determining cognitive impairment based on a gait feature value measured by an accelerometer.

Also, according to the technical solutions of the present disclosure, provided is a cognitive impairment determination method, in which a subject easily and repeatedly performs cognitive impairment determination in everyday life and derives an accurate cognitive impairment determination result that is not affected by a learning effect and not affected by a socio-educational effect.

Description of Drawings

FIG. 1 is a block diagram of a cognitive impairment determination system according to an embodiment.
FIG. 2 is a flowchart of a method of developing a cognitive impairment determination model included in a cognitive impairment determination method, according to an embodiment.

FIG. 3 is a flowchart showing operation 140 of FIG. 2 in detail.

FIG. 4 is a flowchart showing operation 150 of FIG. 2 in detail.

FIG. 5 is a table showing features of datasets of subjects who participated in development of a cognitive impairment determination device .

FIG. 6 is a table showing characteristics of a cognitive impairment determination model according to the present disclosure.

FIGS. 7A to 7C illustrate performance validation results of a cognitive impairment determination model.

FIG. 8 is a table showing a correlation of weighted composite scores of developed models.

FIG. 9 is a block diagram of a cognitive impairment determination device according to an embodiment.

FIG. 10 is a block diagram showing a configuration of a cognitive impairment determination device according to another embodiment.

FIG. 11 is a flowchart of a cognitive impairment determination method according to an embodiment.

Best Mode

The present disclosure relates to various embodiments of a cognitive impairment determination method, device, and computer program product, and the cognitive impairment determination method in which each operation is performed by a processor, includes: determining N subjects who are subject to cognitive impairment determination and obtaining personal data of the subjects; obtaining clinical outcome data for the subjects; obtaining gait data for the subjects, based on gait measurement values measured by using inertia measurement units worn on centers of body mass of the subjects; and developing a cognitive impairment determination model including a plurality of predictors, based on obtained datasets, wherein the cognitive impairment determination model includes gait speeds and step time variability included in the gait data as some of the plurality of predictors.

Mode for Invention

Hereinafter, embodiments of the present disclosure will be described in detail with reference to the accompanying drawings such that one of ordinary skill in the art may easily implement the present disclosure. However, the present disclosure may be implemented in various different forms and is not limited to an embodiment described herein. Also, in the drawings, parts irrelevant to the description are omitted in order to clearly describe the present disclosure, and like reference numerals designate like elements throughout the specification.

In the following embodiments, the terms "first" and "second" are not used in a limited sense and are used to distinguish one component from another component.

In the following embodiments, an expression used in the singular encompasses the expression of the plural, unless it has a clearly different meaning in the context.

In the following embodiments, it will be further understood that the terms "comprise" and/or "comprising" used herein specify the presence of stated features or components, but do not preclude the presence or addition of one or more other features or components.

It will be understood that when a layer, region, or element is referred to as being "formed on" another layer, area, or element, it can be directly or indirectly formed on the other layer, region, or element. That is, for example, intervening layers, regions, or elements may be present.

In the drawings, for convenience of description, sizes of components may be exaggerated or reduced. For example, because shapes, sizes, and thicknesses of components in the drawings are arbitrarily illustrated for convenience of explanation, the present disclosure is not necessarily limited thereto.

According to embodiments, an x-axis, a y-axis, and a z-axis are not limited to three axes on an orthogonal coordinate system, but may be interpreted in a broad sense including the three axes. For example, the x-axis, the y-axis, and the z-axis may be perpendicular to one another, or may represent different directions that are not perpendicular to one another.

When a certain embodiment may be implemented differently, a specific process order or operations may be performed differently from the described order. For example, two consecutively described processes or operations may be performed substantially at the same time or performed in an order opposite to the described order.

Hereinafter, the present disclosure will be described in detail with reference to accompanying drawings.

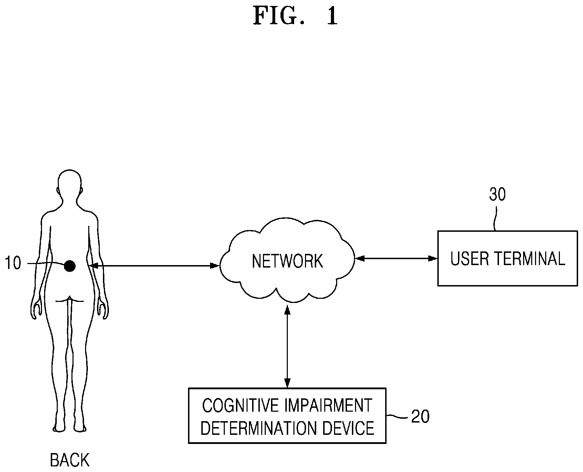

FIG. 1 is a block diagram of a cognitive impairment determination system according to an embodiment.

A cognitive impairment determination system according to an embodiment may include an inertia measurement unit 10, a cognitive impairment determination device 20, and a user terminal 30.

The inertia measurement unit 10 is a device for measuring acceleration and angular velocity and may be attached to a subject to measure tri-axial acceleration, tri-axial angular velocity, and the like through movement of the subject. In other words, the inertia measurement unit 10 may be a device for measuring gait features in space and time.

The inertia measurement unit 10 may be mounted on the centers of body mass of the subject. This is because when the inertia measurement unit 10 is attached to the centers of body mass, most accurate and reliable gait features may be measured. The inertia measurement unit 10 may be a tri-axial accelerometer attached to a person's waist by using a predetermined adhesive material. The inertia measurement unit 10 may transmit a measured value in the form of a signal to the cognitive impairment determination device 20 through a network.

The cognitive impairment determination device 20 may receive signals from the inertia measurement unit 10, receive data from the user terminal 30 or receive data directly from a user, perform cognitive impairment determination based on received values, and provide a result thereof to the user. The cognitive impairment determination device 20 may be implemented as a computer device or a plurality of computer devices, which provide a command, code, file, content, and service by communicating through the network.

According to a selective embodiment, the cognitive impairment determination device 20 may be in the form of a program or application downloaded to the user terminal 30 or the like or may be in the form of a server that provides a cognitive impairment determination method on a web. Hereinafter, a device, server, terminal, application, program, and recording medium storing an application/program, which perform the cognitive impairment determination method of the present disclosure will all be collectively referred to as the cognitive impairment determination device 20.

The user terminal 30 may be a mobile or non-mobile computing device, such as a smartphone, a tablet computer, a personal computer (PC), a wearable smart device, or a health care-dedicated smart device. The user terminal 30 may include any types of devices that communicate with another device through the network. The user terminal 30 may receive a result from the cognitive impairment determination device 20 and transmit data of the subject to the cognitive impairment determination device 20.

According to a selective embodiment of the present disclosure, the user terminal 30 and the cognitive impairment determination device 20 may be integrated with each other. In this case, the cognitive impairment determination device 20 may be installed in the user terminal 30 in the form of an application.

According to another selective embodiment, the user terminal 30, the cognitive impairment determination device 20, and the inertia measurement unit 10 may be integrated with each other. In this case, the inertia measurement unit 10 may be embedded in the user terminal 30 and the cognitive impairment determination device 20 may be installed in the user terminal 30 in the form of an application.

According to another selective embodiment, the user terminal 30 and the inertia measurement unit 10 may be integrated with each other, and the cognitive impairment determination device 20 may receive a value measured by the user terminal 30.

According to another selective embodiment, the system of FIG. 1 may further include a medical service providing server (not shown). A medical service providing server may be a computing device that communicates with another device through the network. The medical service providing server may receive a result from the cognitive impairment determination device 20 and transmit data of the subject to the cognitive impairment determination device 20.

The network may include a local area network (LAN), a wide area network (WAN), a value added network (VAN), a mobile radio communication network, a satellite communication network, or a combination thereof. The network is a data communication network having a comprehensive meaning, which enables seamless communication between network entities shown in FIG. 1, and includes wired Internet, wireless Internet, and a mobile radio communication network. Examples of wireless communication may include wireless LAN (Wi-Fi), Bluetooth, Bluetooth low energy, ZigBee, Wi-Fi direct (WFD), ultra wideband (UWB), infrared data association (IrDA), and near field communication (NFC), but are not limited thereto.

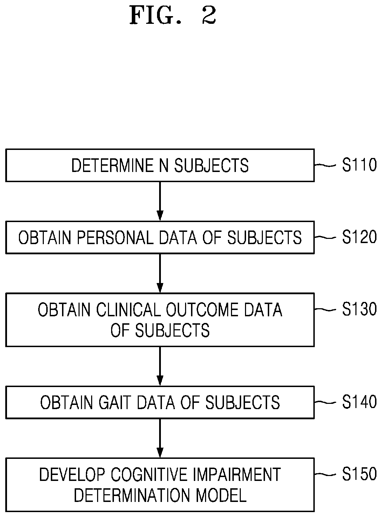

FIG. 2 is a flowchart of a method of developing a cognitive impairment determination model included in a cognitive impairment determination method, according to an embodiment. Each operation shown in FIG. 2 may be performed by a cognitive impairment determination device, in particular, may be performed by a processor included in the cognitive impairment determination device.

First, the cognitive impairment determination device 20 determines N subjects. Here, the N subjects may be determined by criteria stored in the cognitive impairment determination device 20.

Then, the cognitive impairment determination device 20 obtains personal data of the determined subjects. Here, the personal data may be directly input into the cognitive impairment determination device 20 or may be obtained by receiving the same from the user terminal 30 or the medical service providing server. Here, the personal data refers to demographic and anthropometric data of the subjects. According to an embodiment, the personal data may include ages, sexes, educations, heights, weights, presence of lower limb arthritis, and mini mental state examination (MMSE) results (scores) of the subject. Here, lower limb arthritis may include, for example, osteoarthritis, rheumatoid arthritis, or post-traumatic arthritis. Meanwhile, the personal data may further include foot lengths.

Then, the cognitive impairment determination device 20 obtains clinical outcome data of the subjects. The clinical outcome data may be directly input into the cognitive impairment determination device 20 or may be obtained by receiving the same from the user terminal 30 or the medical service providing server. Here, the clinical outcome data may be whether or not the subject is diagnosed with cognitive impairment. When a cognitive disorder (CD) group is defined as a group including subjects diagnosed with cognitive impairment and a cognitively normal (CN) group is defined as a group including subjects not diagnosed with cognitive impairment, after diagnosing cognitive impairment of subjects according to pre-set diagnostic criteria, the subjects diagnosed with cognitive impairment may be included in the CD group and the subjects not diagnosed with cognitive impairment may be included in the CN group. The clinical outcome data may be obtained by inputting a name of a group diagnosed for the subject as clinical outcome data.

Then, the cognitive impairment determination device 20 obtains gait data of the subjects. The cognitive impairment determination device receives a tri-axial acceleration signal and a tri-axial angular velocity signal measured by the inertia measurement unit 10 and processes the same to obtain the gait data. The gait data may include a cadence, a step time, a gait speed, a step length, step time variability, and step time asymmetry.

Then, the cognitive impairment determination device 20 develops a cognitive impairment determination model, based on obtained datasets. The cognitive impairment determination device 20 develops the cognitive impairment determination model through logistic regression analysis and may evaluate diagnostic performance of a comparative model and a model developed through the clinical outcome data.

Meanwhile, in addition to FIG. 2, the cognitive impairment determination device 20 may determine whether a determination target has cognitive impairment by using the developed cognitive impairment determination model and indicate the same to the user.

Hereinafter, each operation will be described in more detail.

In operation 110, N subjects are determined from a population including total M participants.

First, the device determines the population, based on first criteria. Here, the first criteria may be based on a first study. In detail, the total M participants in the first study may be the population and N people from the population may be determined as the subjects. Here, the first study may be a nationwide population-based cohort study for elderly Koreans. According to an embodiment, the first study may be Korean Longitudinal Study on Cognitive Aging and Dementia (KLOSCAD), and in particular, the first study may be "Overview of the Korean longitudinal study on cognitive aging and dementia" (J. W. Han, T. H. Kim, K. P. Kwak, K. Kim, B. J. Kim, S. G. Kim, J. L. Kim, T. H. Kim, S. W. Moon, and J. Y. Park, Psychiatry investigation, vol. 15, no. 8, pp. 767, 2018). However, the present disclosure is not limited thereto and a subject may be changed according to another study or criterion. For example, the device may form a population of total 6818 community-dwelling Koreans aged 60 years or older who are randomly selected using residential lists from 30 regions across the country, based on the first study.

Then, the device determines the N subjects from the determined population, based on second criteria. Here, the second criteria may be criteria for excluding people with a medical history that may affect gait characteristics. In detail, among the participants in the population, people excluding participants with medical history that may affect gait characteristics, based on medical records, may be determined as the N subjects. In detail, among the M participants in the population, N community-dwelling Koreans aged 60 years or older excluding participants with histories of stroke, Parkinson's disease, or movement disorder, histories of unilateral or bilateral knee or hip arthroplasty, histories of knee, hip, or ankle joint surgery, and spinal disease histories such as spinal stenosis, scoliosis, spondylolysis, or spondylolisthesis, may be determined as the subjects.

In operation 120, the personal data of the subjects is obtained.

The device may input demographic and anthropometric data of the subjects as the personal data. For example, skilled researchers may collect the demographic and anthropometric data described above from each subject and input the same to the device as the personal data. Here, the personal data is demographic and anthropometric data of each subject and may include an age, a sex, educations, a height, a weight, and the presence of lower limb arthritis of each subject. Here, the lower limb arthritis may include, for example, osteoarthritis, rheumatoid arthritis, or post-traumatic arthritis. The personal data may further include a foot length.

Also, the device may input, as the personal data, various neuropsychological test results of the subjects. For example, a neuropsychologist or skilled research nurse may conduct a Korean version of a neurocognitive test (Consortium to Establish a Registry for Alzheimer's Disease Assessment Packet (CERAD-K)), a neuropsychological assessment battery, a digit span test, a frontal assessment battery, and MMSE for each subject, and then input results or scores thereof to the device as the personal data.

In operation 130, the device obtains the clinical outcome data of the subjects.

The device may input, as the clinical outcome data, results of the subjects being diagnosed with cognitive impairment according to pre-set diagnostic criteria.

For example, a geriatric psychiatrist or geriatrician may meet with the subject, obtain a detailed medical history, and perform a laboratory test and a physical and neurological examination. Then, a panel including geriatric psychiatrists diagnoses the subject according to the pre-set diagnostic criteria. Here, the diagnostic criteria are the first criteria for dementia and the second criteria for mild cognitive impairment. Meanwhile, Alzheimer's disease may be diagnosed according to third criteria, and dementia with Lewy bodies may be diagnosed according to fourth criteria. Here, the first criteria may be criteria from the fourth edition of the Diagnostic and Statistical Manual of Mental Disorder, the second criteria may be consensus criteria of the International Working Group on Mild Cognitive Impairment, the third criteria may be consensus criteria of the National Institute of Neurological and Communicative Disorders, the Institute of Stroke, and the Association for Alzheimer's Disease and Related Disorders, and the fourth criteria may be consensus criteria proposed by McKeith IG, Dickson D, Lowe J, et al., Diagnosis and management of dementia with Lewy bodies, third report of the DLB consortium, Neurology 2005; 65:1863-1872. However, the first to fourth criteria are not limited thereto and any other criteria that have public confidence may be applied.

Then, the subjects diagnosed with dementia and mild cognitive impairment may be classified into the CD group and the subjects who function independently in the community and do not show any symptoms of cognitive impairment in an objective neuropsychological evaluation may be classified into the CN group. In this case, the obtaining of the clinical outcome data may indicate receiving results of the subjects being classified into the CD group or the CN group according to the above-described criteria.

In operation 140, the device obtains the gait data of the subjects.

The device receives or is input with a gait measurement value from an inertia measurement unit attached to the subject and generates the gait data based on the input gait measurement value.

The gait data may be generated based on signals measured from the inertia measurement unit. The gait data may include a cadence, a step time, a gait speed, a step length, step time variability, and step time asymmetry.

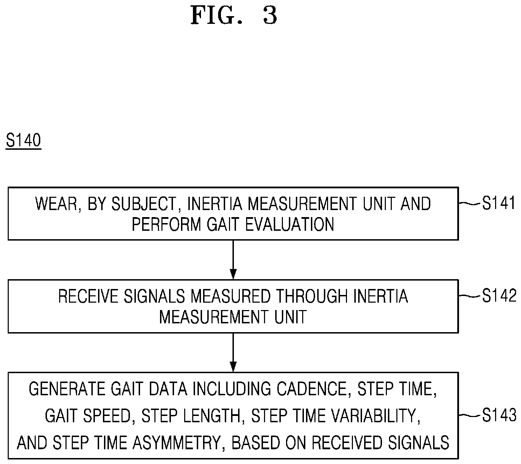

FIG. 3 is a flowchart showing operation 140 of FIG. 2 in detail. Hereinafter, operation 140 will be described in detail with reference to FIG. 3.

Referring to FIG. 3, in operation 141, the subject wears the inertia measurement unit 10 and performs a gait evaluation.

According to an embodiment, the inertia measurement unit 10 may include a digital tri-axial accelerometer sensor and a gyroscope. For example, FITMETERO from FitLife Inc., which is a 14 g smooth-edged hexahedron having a size of $3.5 \times 3.5 \times 1.3$ cm, or ActiGraph® from SMD solution, which is a 17 g smooth-edged hexahedron having a size of $3.0 \times 4.0 \times 1.0$ cm, may be used as an inertia measurement unit. Such an inertia measurement unit may measure tri-axial acceleration up to $\pm 8$ g (resolution 0.004 g/0.00024 g) and tri-axial angular velocity up to $\pm 1,000$/s (resolution 0.03/s) at a sample rate of 250 Hz. However, the present disclosure is not limited thereto and a smartphone or smart medical device embedded with a digital accelerometer sensor and a gyroscope may be used as the inertia measurement unit 10.

The gait evaluation of the subject is performed while the inertia measurement unit 10 is attached to the centers of body mass of the subject.

The inertia measurement unit 10 may be fixed to the 3rd to 4th lumbar vertebrae of the back corresponding to the center of body mass of the subject by using a predetermined adhesive or an elastic band.

The gait evaluation is conducted by having the subjects, to which the inertia measurement unit 10 is attached, comfortably walk a walking section back and forth three times at a self-selected speed. Here, the walking section may include a 14 m even straight walking path and a route may be designed to turn after passing a 14 m line. However, a gait evaluation method and the walking section are not limited to the above and may be variously modified.

In operation 142, the cognitive impairment determination device 20 receives signals measured by using the inertia measurement unit 10.

The inertia measurement unit 10 transmits the signals measured while the subject walks the walking section, to the cognitive impairment determination device 20. Here, the signals include various gait measurement values, for example, tri-axial acceleration and tri-axial angular velocity.

According to an embodiment, the cognitive impairment determination device 20 may preprocess the received signals. In detail, the cognitive impairment determination device 20 may remove, from the obtained signals, signals corresponding to pre-set lengths of the front and rear of the walking section in order to minimize an acceleration effect of the subject and measure normal walking. For example, the cognitive impairment determination device 20 may remove signals corresponding to 2 m long walking sections before a reference point and before the subject turns, and process only signals corresponding to remaining intermediate 10 m walking section as valid signals.

In operation 143, the cognitive impairment determination device 20 generates the gait data based on the obtained signals.

The cognitive impairment determination device 20 processes the signals and identifies each step. The cognitive impairment determination device 20 obtains the gait data based on the signals obtained from the inertia measurement unit 10 and the previously obtained personal data of the subject. Here, the gait data may include a cadence, a step time, a gait speed, a step length, step time variability, and step time asymmetry.

A cadence denotes the number of steps per minute, i.e., a walking rate, and a unit may be steps/min. A cadence may be derived by calculating the number of steps per minute when the device receives an acceleration signal of the subject for a certain period of time and defines a section between peaks of the acceleration signal as steps.

A unit of a step time is seconds, and the step time is defined as a duration of each step from a first contact of one foot to a first contact of an opposite foot. A step time is estimated as a value obtained by multiplying an inverse credence by 60.

A unit of a gait speed is cm/s and the gait speed may be defined as a distance a body moves on a flatland in cm per second. A gait speed may be derived through a mathematical linear regression equation including a roll angle, a yaw angle, and a weight as walk factors. For example, according to Equation 1, a linear regression equation of a first model includes, as walk factors for estimating a gait speed, an age, a cadence, vertical height displacement (VHD), a foot length, a weight, a roll angle, and a yaw angle, and may have a coefficient corresponding to each walk factor. Here, the VHD is an average of differences between maximum and minimum vertical heights of the center of body mass within one gait cycle, and may be calculated from data from an inertia measurement unit. A foot length is an average length of both feet and may be obtained from personal data. A roll angle and a yaw angle of the center of body mass may reflect angular motion of a torso and limbs. The roll angle and the yaw angle are calculated from data of the inertia measurement unit, and values thereof may be re-oriented into Cartesian coordinates.

Gait Speed [Model 1] = -106.0 - 0.328 × Age + 1.10 × Cadence + 10.1 × VHD + 3.29 × Foot length - 0.115 × Weight + 1.01 × Roll angle + 0.647 × Yaw angle          [Equation 1]

However, the present disclosure is not limited to deriving the gait speed according to Equation 1 and the gait speed may also be derived by using various models described in Patent Application No. 10-2021-0194313 filed by the applicant of the present disclosure.

A unit of a step length is cm and the step length may be defined as a distance between a previous heel and a current heel. The step length may be a value obtained by multiplying a gait speed by 60 and then dividing a result by a cadence, as represented by Equation 2 below.

[Equation 2]

$$step\ length\ (cm/step) = \frac{gait\ speed \times 60}{cadence}$$

A unit of step time variability is % and in the present specification, the step time variability may be expressed as a coefficient of variance step time (CV step time). The step time variability may be defined as an indicator of how constant a gait cycle of left and right feet is maintained. The CV step time may be derived as shown in Equation 3 below. The CV step time may be derived by using a model described in Patent Application No. 10-2016-0124602 (Tri-Axial Accelerometer-based Method for Detecting Gait Pattern and Gait Event) filed by the applicant of the present disclosure. In detail, a CV step time for a left step is obtained by using standard deviation and a mean of a step time of the left step and a CV step time for a right step is obtained by using standard deviation and a mean of a step time of the right step, and then a final CV step time may be derived by dividing the sum of the CV step times of the left and right steps by 2.

[Equation 3]

$$CV\ Step\ Time\ (\%) = \left(\frac{Standard\ Deviation\ of\ Step\ Time}{Mean\ of\ Step\ Time}\right) \times 100$$

In the present specification, step time variability may have a same value as temporal gait variability. In general, gait variability may include temporal gait variability and spatial gait variability. The temporal gait variability is more sensitive to initial abnormal gait variation and has higher reliability than the spatial gait variability. Accordingly, by using the step time variability, which corresponds to the temporal gait variability, as an independent variable of a cognitive impairment determination model, a reliable cognitive impairment determination model may be developed.

A unit of step time asymmetry is seconds (sec) or milliseconds (ms) and the step time asymmetry may be defined as an absolute difference between a mean value of a step time for a left step and a mean value of a step time for a right step.

Referring back to FIG. 2, the cognitive impairment determination model is developed based on datasets obtained in operation 150.

The cognitive impairment determination model may be a gait data-based algorithm and may include a plurality of predictors. In detail, the cognitive impairment determination model may include the gait speed and the step time variability as some of the predictors.

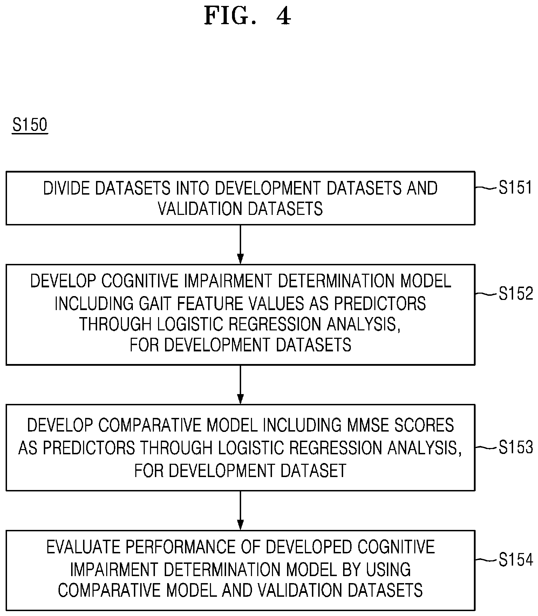

FIG. 4 is a flowchart showing operation 150 of FIG. 2 in detail. Hereinafter, a method of developing the cognitive impairment determination model will be described in more detail with reference to FIG. 4.

Referring to FIG. 4, in operation 151, the cognitive impairment determination device 20 divides total obtained datasets into development datasets and validation datasets.

Here, a dataset denotes a set of all pieces of data including personal data, clinical outcome data, and gait data of each subject.

The cognitive impairment determination device 20 may randomly divide the datasets. However, the present disclosure is not limited thereto and the datasets may be divided so that a same ratio of datasets of subjects in the CD group and datasets of subjects in the CN group are distributed to both sets. For example, both the development datasets and the validation datasets may be obtained to include about 17% of the datasets of subjects in the CD group. Here, the CD group and CN group are obtained by classifying the subjects who are owners of the datasets, according to diagnostic criteria, such that the subjects diagnosed with dementia, mild cognitive impairment, Alzheimer's disease, or dementia caused by Lewy bodies are classified into the CD group, and the subjects who function independently in the community and do not show any symptoms of cognitive impairment in an objective neuropsychological evaluation are classified into the CN group. Information related to the CD group and CN group may be obtained from pre-obtained clinical outcome data.

The development datasets may be obtained to include a larger number of datasets than the validation datasets. For example, the total datasets may be divided into 80% of development datasets and 20% of validation datasets.

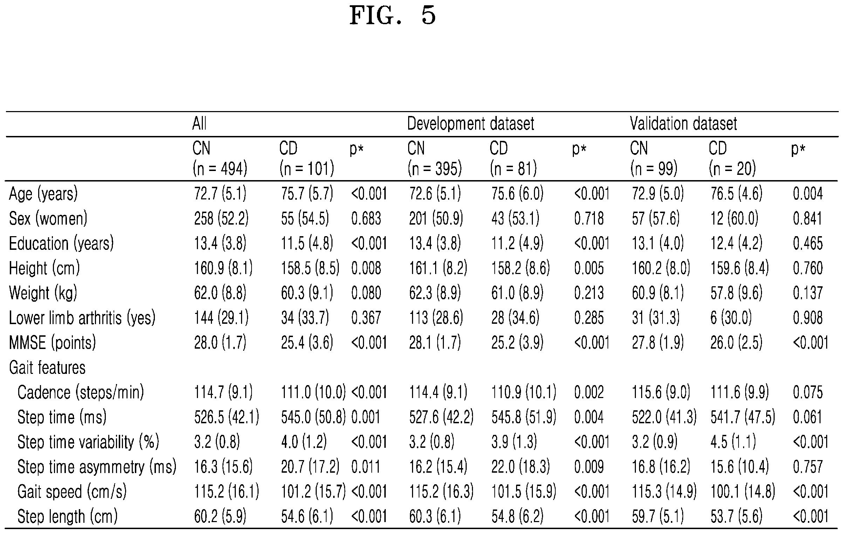

FIG. 5 is a table showing features of the datasets of the subjects who participated in development of the cognitive impairment determination device 20.

Referring to FIG. 5, CN indicates datasets of subjects included in the CN group, and CD indicates datasets of subjects included in the CD group. All indicates all datasets, Development dataset indicates the development datasets, and Validation dataset indicates the validation datasets.

Among values included in the table of FIG. 5, a continuous variable is expressed in an average (standard deviation) and a categorical variable is expressed in a number (percentage). The continuous variables are compared using a Student's t-test, and the categorical variables are compared using a chi-squared test to obtain a p-value (p*).

Referring to FIG. 5, for total 595 subjects, 101 subjects (17%) are included in the CD group, and the remaining 494 subjects are included in the CN group. In detail, according to the obtained clinical outcome data, among the 101 subjects included in the CD group, 94 subjects are diagnosed with mild cognitive impairment, 6 subjects are diagnosed with dementia due to Alzheimer's disease, and 1 subject is diagnosed with dementia due to Lewy bodies.

Looking at personal data of the subjects included in the CD group, it is determined that the subjects are older, have less educations, are shorter, and have lower MMSE scores than those in the CN group. Also, looking at gait data of the subjects included in the CD group, it is determined that the subjects have lower cadences, longer step times, greater step time variability, greater step time asymmetry, slower gait speeds, and shorter step lengths than those in the CN group.

In operation 152, the cognitive impairment determination device 20 develops the cognitive impairment determination model using logistic regression analysis for the development datasets.

The cognitive impairment determination model may be a regression model for determining whether a subject has cognitive impairment by using a measured value of the inertia measurement unit 10. Here, the determining of whether the subject has cognitive impairment is performed by estimating likelihood that a determination target will be diagnosed with dementia or mild cognitive impairment according to the above-described diagnostic criteria.

According to an embodiment, a regression-based model for determining cognitive impairment is developed by using a binary logistic regression analysis model applying forward selection.

The binary logistic regression analysis model is a model to which a plurality of independent variables are input so as to determine which variable among the input independent variables is better at determining a dependent variable. The forward selection is a method of adding a variable with best explanatory power among the input independent variables, and when the input independent variable satisfies a level of statistical significance, the model sequentially adopts the corresponding independent variable.

Here, the dependent variable denotes whether cognitive impairment is present and a dichotomous result of presence of cognitive impairment or no cognitive impairment is derived.

Here, the independent variables used are step time variability, a gait speed, an age, a sex, educations, a height, and presence of lower limb arthritis. The cognitive impairment determination device 20 stores the personal data and gait data of the subject. The personal data includes an age, a sex, educations, a height, a weight, presence of lower limb arthritis, and an MMSE result, and the gait data includes a cadence, a step time, a gait speed, a step length, step time variability, and step time asymmetry.

The cognitive impairment determination device 20 may use, as the independent variables, the step time variability, the gait speed, the age, the sex, the educations, the height, and the presence of lower limb arthritis, which are selected from such variables according to criteria. The criteria for selecting an independent variable may be selected by determining whether the independent variable has a significant influence on cognitive impairment determination.

For example, it has been verified through various existing studies that an age, a sex, and educations are variables that influence the cognitive impairment determination.

Also, the step time variability and the gait speed are important factors of the cognitive impairment determination. In detail, according to existing neuroimaging studies, such as Blumen HM, Brown LL, Habeck C, et al., Gray matter volume covariance patterns associated with gait speed in older adults: a multi-cohort MRI study, Brain imaging and behavior 2019;

13:446-460, the gait speed is related to the volume of brainstem, cerebellum, primary and supplementary motor cortex, and prefrontal cortex areas involved in information processing speed and executive functions. Meanwhile, according to existing neuroimaging studies, such as Tian Q, Chastan N, Bair W-N, Resnick SM, Ferrucci L, Studenski SA, The brain map of gait variability in aging, cognitive impairment, and dementia-a systematic review, Neuroscience & Biobehavioral Reviews 2017; 74:149-162, the gait variability is related to the volume of primary sensorimotor cortex, hippocampus, anterior cingulate cortex, basal ganglia, and posterior thalamic radiation involved in overall gait, memory, and executive functions.

In addition, the height has a strong correlation with the step time variability and the gait speed, and the presence of lower limb arthritis also has a strong correlation with the step time variability and the gait speed.

The device may input the independent variables selected based on such criteria to the binary logistic regression model using the forward selection, select, as the predictors, the independent variables that have a significant influence on deriving the dependent variable, and develop the cognitive impairment determination model including the selected predictors. According to an embodiment of the present disclosure, the developed cognitive impairment determination model may include the gait speed, the step time variability, and the educations as significant predictors. In other words, the cognitive impairment determination model according to an embodiment of the present disclosure is a model that includes a gait measurement value as the predictor when determining whether a subject has cognitive impairment.

Equation 4 is a mathematical linear regression equation of the developed cognitive impairment determination model. The gait speed is extracted from the gait data and the step time variability is also extracted from the gait data. The education is extracted from the personal data.

$$\text{--> } 2.286 - 0.040 \times \text{Gait Speed} + 0.535 \times \text{Step Time Variability} - 0.115 \times \text{Education} \qquad \text{[Equation 4]}$$

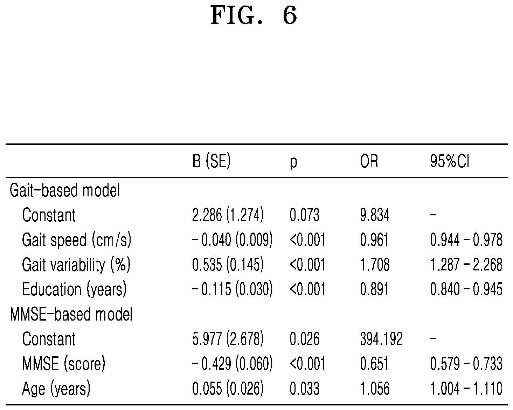

FIG. 6 is a table showing characteristics of the cognitive impairment determination model according to the present disclosure.

Referring to Equation 4 and FIG. 6, the cognitive impairment determination model includes a predictor and a regression coefficient (B).

The predictors are the gait speed, the step time variability (indicated as gait variability in FIG. 6, and the educations, and the predictors may have respective regression coefficients (B in FIG. 6). For example, the regression coefficient of the gait speed is -0.040, the regression coefficient of the step time variability is 0.535, and the regression coefficient of the educations is -0.115. Each regression coefficient denotes a relative measure of discriminative power for the predictors and may indicate the power of how well the predictors included in the model discriminate the dependent variable. The larger the regression coefficient, the more influence the corresponding predictor has on determining the dependent variable. Among the predictors included in the cognitive impairment determination model of the present disclosure, the step time variability corresponds to a variable with the greatest explanatory power. Meanwhile, 2.286 in Equation 4 corresponds to a regression constant (constant in FIG. 6).

As shown in a p-value (p) in FIG. 6, the gait speed, the step time variability, and the educations have low p-values (p) values ($p < 0.05$), and thus, it is determined again that they are statistically significant values.

In FIG. 6, OR denotes an odds ratio and indicates a possibility of how much the dependent variable changes when the independent variable input into the model changes. For example, in a case of the gait speed, it is determined that when the gait speed decreases by 1 cm/s, a risk of being determined as having cognitive impairment increases by 0.961 times. 95% CI denotes a confidence interval and indicates a confidence interval of OR.

In the cognitive impairment determination model represented by Equation 4, an optimal cutoff for determining cognitive impairment may be derived as > - 1.56. Here, the optimal cutoff corresponds to a reference score for determining cognitive impairment. In other words, when the gait speed, step time variability, and educations of the subject are input into the cognitive impairment determination model, a real number is derived, and if the real number is greater than -1.56, the subject may be determined to have cognitive impairment. If the real number derived from the model is less than or equal to -1.56, the subject may be determined to not have cognitive impairment.

For reference, the optimal cutoff may be derived through bootstrap sampling, wherein sensitivity is $72.0 \pm 6.6\%$ and specificity is $74.4 \pm 5.9\%$.

In operation 153, the cognitive impairment determination device 20 develops a comparative model using logistic regression analysis for the development datasets.

The comparative model may be a regression model for determining whether a subject has cognitive impairment by using a MMSE score instead of a measurement value of an inertia measurement unit. The comparative model may be derived to validate the cognitive impairment determination model. A method of deriving the comparative model is similar to the method of deriving the cognitive impairment determination model described above, and thus, redundant description will be omitted.

An MMSE score and an age may be used as independent variables to derive the comparative model. The comparative model derived in as such may include an MMSE score and an age as significant predictors. In other words, the comparative model is a model that includes an MMSE score as a predictor when determining whether a subject has cognitive impairment.

Equation 5 is the mathematical linear regression equation of the comparative model. An MMSE score is extracted from the personal data and an age is also extracted from the personal data. For reference, an optimal cutoff for the comparative model to determine cognitive impairment is derived to be > -1.16, sensitivity is derived to be $56.7 \pm 3.2\%$, and specificity is derived to be $88.3 \pm 1.4\%$.

[Equation 5]

$$5.977 - 0.429 \times MMSE\ score + 0.055 \times age$$

In operation 154, the cognitive impairment determination device 20 validates the developed cognitive impairment determination model.

The cognitive impairment determination model may be validated based on statistical criteria, may be validated by the comparative model, or may be validated through the validation datasets.

First, the cognitive impairment determination device 20 may test suitability of the model, based on the statistical criteria.

In detail, the cognitive impairment determination device 20 may evaluate statistical goodness of fit of the model by using a Hosmer-Lemeshow test with $p > 0.05$ and algorithm explanatory power (Nagelkerke R2 index). A regression coefficient may be interpreted as a relative weighted value of an independent variable to a dependent variable. Then, a weighted composite score may be calculated by using a logit score to which the weighted value of the regression coefficient of each logistic regression model is applied.

Also, the cognitive impairment determination device 20 may validate performance of the cognitive impairment determination model through an area under the curve (AUC). Here, the performance denotes cognitive impairment determination performance or diagnostic performance, and indicates how accurately cognitive impairment is determined.

According to an embodiment, the cognitive impairment determination device 20 may validate the performance by deriving and comparing AUC of the cognitive impairment determination model and comparative model. In detail, the cognitive impairment determination device 20 may validate the performance of the cognitive impairment determination model by deriving and comparing receiver operating characteristics (ROC) curves of both models, for the development datasets and the validation datasets. More specifically, the cognitive impairment determination device 20 may generate ROC curves for each of the two models and compare the AUC of the two models by using a method proposed in Hanley JA, McNeil BJ, A method of comparing the areas under receiver operating characteristic curves derived from the same cases, Radiology 1983; 148:839-843, thereby evaluating the determination performance or diagnostic performance of the cognitive impairment determination model.

According to an embodiment, the cognitive impairment determination device 20 may validate the performance by deriving and comparing the AUC of both models for the development datasets. First, the cognitive impairment determination device 20 may estimate an optimal cutoff, sensitivity, and specificity of a weighted composite score by using a bootstrap sampling estimation method. In this case, 80% of the development datasets may be randomly selected in each run. The cognitive impairment determination device 20 may obtain a bootstrap estimate of the AUC, the optimal cutoff, the sensitivity, and the specificity by combining results of 100 runs. Then, validation may be performed by deriving and comparing bootstrap-estimated average AUC between the cognitive impairment determination model and the comparative model.

According to another embodiment, the cognitive impairment determination device 20 may compare the AUC for the validation datasets with the AUC for the development datasets. In detail, the cognitive impairment determination device 20 may determine an overfitting possibility by comparing the AUC of the cognitive impairment determination model obtained from the validation datasets with the AUC of the cognitive impairment determination model obtained from the development datasets.

Also, the cognitive impairment determination device 20 may perform validation by comparing the AUC of both models obtained from the validation datasets.

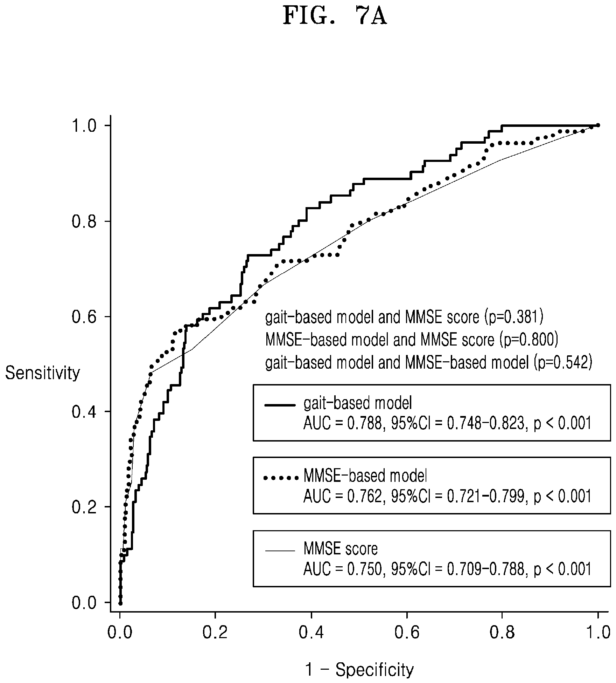

FIGS. 7A to 7C illustrate performance validation results of the cognitive impairment determination model. Hereinafter, the performance validation results of the cognitive impairment determination model will be described with reference to FIGS. 7A to 7C. The performance validation results described below are for reference only, and specific result values may vary depending on conditions, such as a configuration of a dataset and a size of a variable. Meanwhile, statistical significance used in the present specification is a two-tailed test and a p-value may be set to $p < 0.05$.

First, a cognitive impairment determination model including a gait data-based algorithm (gait-based model) shows adequate statistical goodness of fit. In detail, the adequate statistical goodness of fit is shown as Hosmer-Lemeshow test result $\chi^2 = 4.16$, df = 8, p = 0.843, and algorithm explanatory power = 0.243 are derived. In addition, algorithm classification accuracy (%) is calculated to be highly accurate at 83.8%.

For reference, a comparative model including an MMSE score-based algorithm (MMSE-based model) also shows adequate statistical goodness of fit. In detail, the adequate statistical goodness of fit is shown as Hosmer-Lemeshow test result $\chi^2 = 12.99$, df = 8, p = 0.112, and algorithm explanatory power = 0.271 are derived. In addition, algorithm classification accuracy is calculated to be highly accurate at 86.1%.

Meanwhile, as shown in FIGS. 7A to 7C, the cognitive impairment determination model shows a high AUC value, confirming that the determination performance or diagnostic performance is good. An AUC is a numerical value for evaluating performance of a model, and an AUC value of 0.7 to 0.8 is considered fair, 0.8 to 0.9 is considered good, and 0.9 to 1.0 is considered excellent.

Referring to FIG. 7A, results of obtaining the AUC of the cognitive impairment determination model and the AUC of the comparative model within the development datasets are shown. In detail, the cognitive impairment determination model has the AUC of 0.788 in the development datasets, wherein a standard error [SE] is 0.027, 95% CI is 0.748 to 0.823, and a p value is p < 0.001. The comparative model has the AUC of 0.762 in the development datasets, wherein a standard error [SE] is 0.033, 95% CI is 0.721 to 0.799, and a p value is p < 0.001. As such, the AUC of the cognitive impairment determination model including the gait data-based algorithm (gait-based model) is greater than the AUC of the comparative model including the MMSE score-based algorithm (MMSE-based model), and thus, it is verified that the cognitive impairment determination model has excellent performance.

Next, the AUCs for the two models are compared for 100 samples extracted from the development datasets through bootstrap sampling. A bootstrap-estimated average AUC of the cognitive impairment determination model is derived to be 0.787 ± 0.015, and a bootstrap-estimated average AUC of the comparative model is derived to be 0.760 ± 0.016. In this case as well, it is verified that the AUC of the cognitive impairment determination model is greater than that of the comparative model.

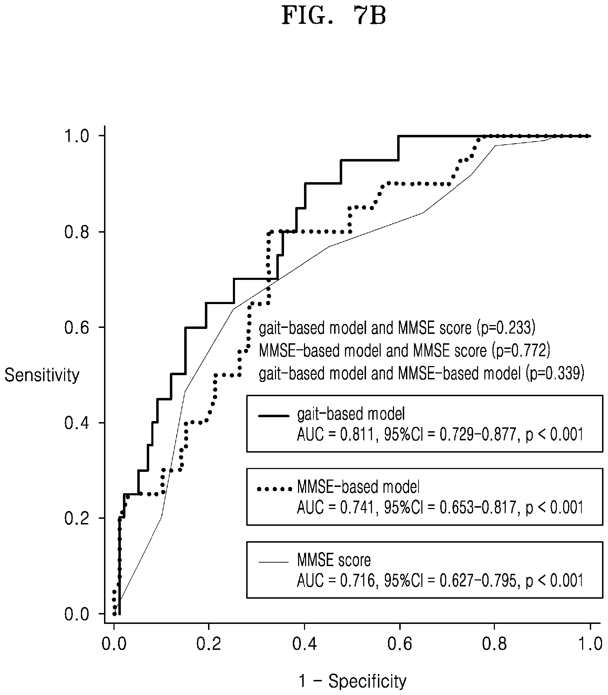

In addition, when the cognitive impairment determination model is applied to independent validation datasets, the determination performance or diagnostic performance is evaluated as good. Referring to FIG. 7B, the AUC of the cognitive impairment determination model at this time is 0.811, SE is 0.046, 95% CI is 0.729 to 0.877, and a p-value is p < 0.001. It is verified that the AUC of the cognitive impairment determination model has a greater value than the AUC of the comparative model.

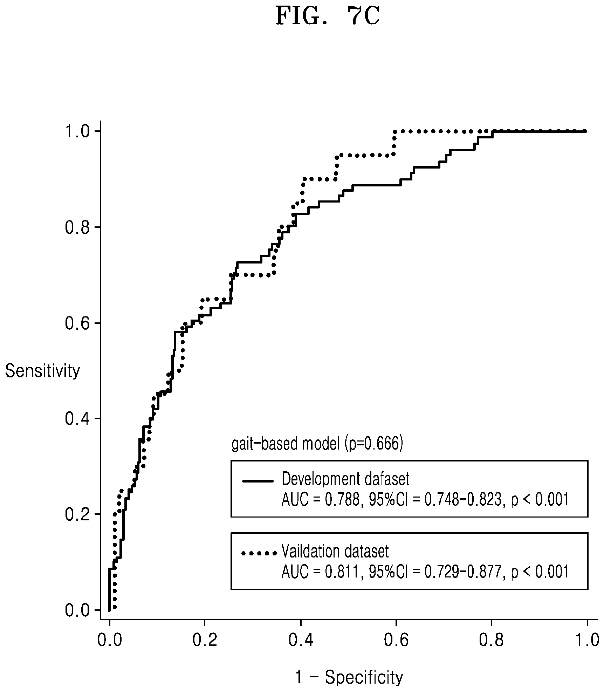

Also, a high AUC may be determined even when the cognitive impairment determination model is applied to each of the validation datasets and the development datasets. Referring to FIG. 7C, when the cognitive impairment determination model is applied to the development datasets, the AUC is determined to be 0.788, and when the cognitive impairment determination model is applied to the validation datasets, the AUC is determined to be 0.811. In other words, it is verified that the cognitive impairment determination model shows similarly high determination performance or diagnostic performance in both datasets.

Meanwhile, according to a selective embodiment, the cognitive impairment determination device may determine that the models have a correlation and have similar performance of determining cognitive impairment despite of a slight difference, by using Pearson's correlation analysis. Correlation analysis may be implemented by using Pearson's correlation analysis for various datasets to analyze correlations between the cognitive impairment determination model, the comparative model, and the weighted composite score of the MMSE score.

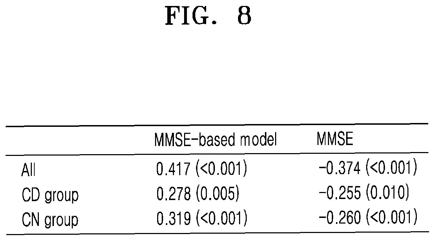

FIG. 8 is a table showing the correlation of the weighted composite scores of the developed models.

Referring to FIG. 8, it is determined that the correlation between the weighted composite scores of the CD group and the CN group for all subjects of the cognitive impairment determination model and the comparative model is very significant. In addition, it is determined that the correlation between the weighted composite scores of the CD group and the CN group for subjects of the cognitive impairment determination model and the MMSE score is also very significant.

In summary, the cognitive impairment determination device according to an embodiment includes a model for determining subjects with cognitive impairment. The model may be developed and validated through gait characteristics obtained based on measurement values obtained through a wearable inertia sensor worn at a center of body mass of a determination target. As determined from validation results, it may be verified that the cognitive impairment determination device shows a high level of determination performance or diagnostic performance. In particular, when comparing the cognitive impairment determination model with the comparative model using MMSE scores that are widely used to determine cognitive impairment clinically and in research, it is determined that the cognitive impairment determination model shows similar or better performance.

According to an embodiment, the cognitive impairment determination model includes the gait speed and the step time variability as significant predictors. Thus, the cognitive impairment determination model according to an embodiment is able to determine all types or various causes of cognitive impairment.

In detail, according to existing neuroimaging studies, the gait speed is related to the volume of brainstem, cerebellum, primary and supplementary motor cortex, and prefrontal cortex areas involved in information processing speed and executive functions. On the other hand, the gait variability is related to the volume of primary sensorimotor cortex, hippocampus, anterior cingulate cortex, basal ganglia, and posterior thalamic radiation involved in overall gait, memory, and executive functions. Accordingly, the cognitive impairment determination model of the present disclosure includes the gait speed and the step time variability as significant variables, and thus is able to successfully determine all causes of cognitive impairment. Alzheimer's disease is the most common type of dementia, accounting for 60 to 80% of dementia cases, followed by vascular dementia and dementia with Lewy bodies/Parkinson's dementia, accounting for 20%. 46% of Alzheimer's disease patients have vascular pathology. Thus, the cognitive impairment determination method according to an embodiment of the present disclosure may be used to screen for dementia regardless of a root cause.

MMSE is widely used to determine cognitive impairment clinically and in research, but the MMSE has a few drawbacks. MMSE is unable to be administered by a determination target alone, has a learning effect when administered repeatedly, and is easily socially and educationally influenced. In addition, MMSE is not adequate for screening for cognitive impairment in an early stage.

However, the cognitive impairment determination method according to an embodiment may be implemented by being installed in a user's terminal as an application, and a gait is analyzed through signals measured as a determination target wears a wearable inertia sensor, and thus, the cognitive impairment determination method may be easily and repeatedly used by an individual in everyday life. In addition, the cognitive impairment determination method is not affected by a learning effect and may be free from socio-educational influences. The cognitive impairment determination method not only determines cognitive impairment, but also continuously monitors the determination target by using used in the real life of the determination target.

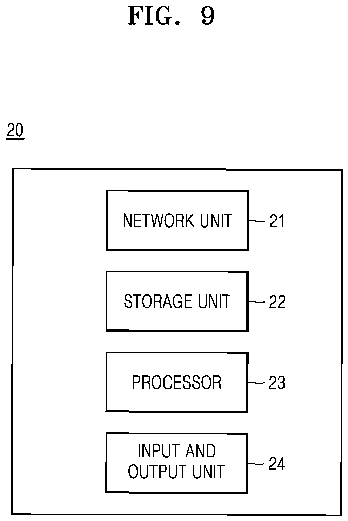

FIG. 9 is a block diagram of the cognitive impairment determination device 20 according to an embodiment.

The cognitive impairment determination device 20 may include a network unit 21, a storage unit 22, a processor 23, and an input and output unit 24. FIG. 9 only illustrates components related to an embodiment, and the cognitive impairment determination device 20 may further include other general-purpose components in addition to the components shown in FIG. 9.

The network unit 21 may include one or more components that enable wired/wireless communication with other nodes. For example, the network unit 21 may include at least one of a short-range wireless communication unit (not shown), a mobile communication unit (not shown), and a broadcast receiver (not shown).

The network unit 21 communicates with the inertia measurement unit 10 to receive gait measurement values, communicates with the user terminal 30 to receive personal data, or communicates with a medical service providing server to receive N subjects determination information and clinical outcome data.

The storage unit 22 is hardware storing various types of data processed in cognitive impairment determination device 20, and may store a program for processes and control by the processor 23.

The storage unit 22 may store the personal data obtained in operation 120 of FIG. 2, the clinical outcome data obtained in operation 130, and the gait data obtained in operation 140.

In addition, the storage unit 22 may store various programs for developing the cognitive impairment determination device 20 in operation 150 of FIG. 2 and various programs used to validate the developed cognitive impairment determination device 20. For example, a program may be executed by using R version 4.2.0 (Foundation for Statistical Computing, Vienna, Austria), the Statistical Package for the Social Sciences for Windows version 20 (International Business Machines Corporation, Armonk, NY), and MedCalc for Windows version 18.11.3 (MedCalc Software, Mariakerke, Belgium), but the present disclosure is not limited thereto.

The storage unit 22 may include a random access memory (RAM) such as a dynamic random access memory (DRAM) or a static random access memory (SRAM), a read-only memory (ROM), an electrically erasable programmable read-only memory (EEPROM), CD-ROM, Blu-ray or another optical disk storage, a hard disk drive (HDD), a solid state drive (SSD), or a flash memory.

The input and output unit 24 may include an input unit including a keypad, a touch screen, or the like, and an output unit including a display, a speaker, or the like, and the input and output unit 24 may be implemented as a touch display.

The input and output unit 24 may receive the personal data of operation 120 or the clinical outcome data of operation 130 of FIG. 2 directly from the user. Also, the input and output unit 24 may provide the user with a cognitive impairment determination result by the cognitive impairment determination device 20 or a determination result that is a dependent variable of the cognitive impairment determination model.

The processor 23 controls overall operations of the cognitive impairment determination device 20. For example, the processor 23 may execute the programs stored in the storage unit 22 to control the input and output unit 24, the network unit 21, and the storage unit 22 in general.

The processor 23 may perform each operation of FIGS. 2, 3, and 4 and control components, in detail, control the network unit 21 to receive measured values, information, and data, generate gait data from the measured values, control the storage unit 22 to store obtained datasets, and execute the programs stored in the storage unit 22 to develop and validate the cognitive impairment determination model by using the datasets, determine cognitive impairment by using the developed cognitive impairment determination model, and provide a result thereof to the user.

The processor 23 may be realized by using at least one of an application-specific integrated circuit (ASIC), a digital signal processor (DSP), a digital signal processing device (DSPD), a programmable logic device (PLD), a field programmable gate array (FPGA), a controller, a micro-controller, a microprocessor, and electric units for performing other functions.

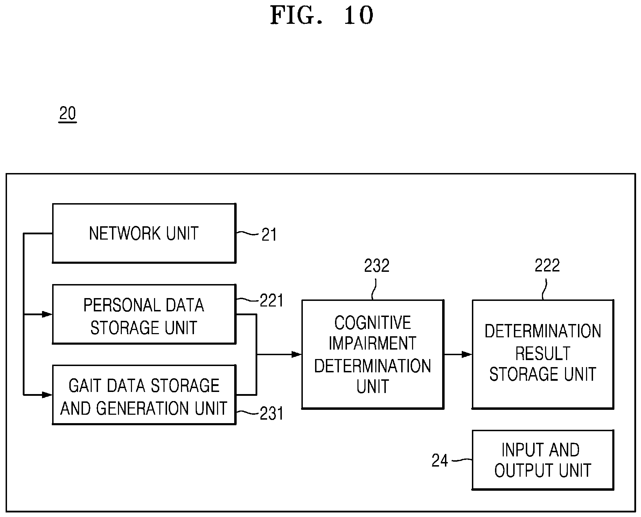

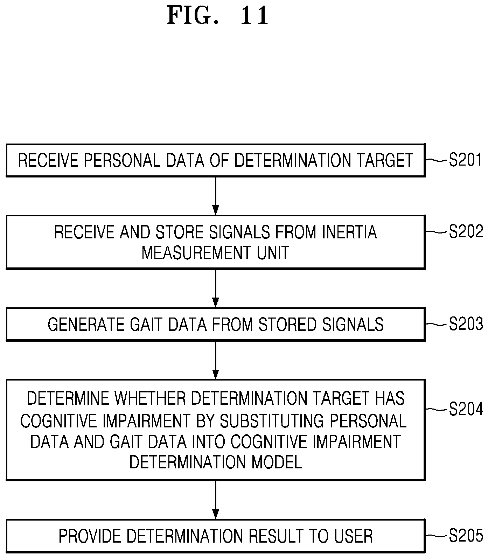

FIG. 10 is a block diagram showing a configuration of the cognitive impairment determination device 20 according to another embodiment. FIG. 11 is a flowchart of the cognitive impairment determination method according to an embodiment. The cognitive impairment determination method using the cognitive impairment determination device 20 will be described in detail with reference to FIGS. 10 and 11.

The cognitive impairment determination device 20 of FIG. 10 includes the network unit 21, a personal data storage unit 221, a gait data storage and generation unit 231, a cognitive impairment determination unit 232, a determination result storage unit 222, and the input and output unit 24.

In operation 201, the cognitive impairment determination device 20 receives personal data of a determination target. The determination target may input the personal data through the input and output unit 24 and the input personal data may be stored in the personal data storage unit 221. Here, the personal data includes educations and may further include an age, a weight, a foot length, or the like.

In operation 202, the cognitive impairment determination device 20 receives signals from the inertia measurement unit 10 attached to the determination target. The determination target attaches the inertia measurement unit 10 to the center of body mass and performs a gait evaluation. The inertia measurement unit 10 transmits signals measured during walking to the cognitive impairment determination device 20. The cognitive impairment determination device 20 stores the signals received through the network unit 21 in the gait data storage and generation unit 231. Here, the signals may be a tri-axial acceleration or tri-axial angular velocity measurement value measured during walking.

In operation 203, the cognitive impairment determination device 20 generates gait data from the stored signals. Here, the gait data may include a gait speed and step time variability. In detail, the gait data storage and generation unit 231 may calculate the gait speed based on the signals and the stored personal data, and calculate the step time variability based on the stored signals.

In operation 204, the cognitive impairment determination device 20 may determine whether the determination target has cognitive impairment by substituting the personal data and the gait data into the cognitive impairment determination model. The cognitive impairment determination unit 232 includes the cognitive impairment determination model developed above. The cognitive impairment determination model is developed by using a binary logistic regression method applying forward selection, is in the form of a linear regression equation, and includes a gait speed, step time variability, and educations as predictors, and each predictor has a pre-set regression coefficient and also includes a pre-set regression constant. The cognitive impairment determination model is a linear regression equation and represented as Equation 6 below. An optimal cutoff, which is a reference score for determining cognitive impairment, in the linear regression equation of Equation 6 may be > -1.56.

$$2.286 - 0.040 \times \text{Gait Speed} + 0.535 \times \text{Step Time Variability} - 0.115 \times \text{Education} \qquad \text{[Equation 6]}$$

The cognitive impairment determination unit 232 inputs the gait speed, the step time variability, and the educations of the determination target into the model as inputs, and obtains a score expressed as a real number as an output. When the obtained score is greater than the optimal cutoff, the cognitive impairment determination unit 232 may determine that the determination target has cognitive impairment. When the obtained score is less than or equal to the optimal cutoff, the cognitive impairment determination unit 232 may determine that the determination target does not have cognitive impairment.

In operation 205, the cognitive impairment determination device 20 provides a determination result to a user. Here, the user may be the determination target, or may be a medical staff or a guardian. The cognitive impairment determination device 20 may directly provide the determination result to the user through the input and output unit 24. However, the present disclosure is not limited thereto, and the determination result may be provided to the guardian or the medical staff by transmitting the determination result to another user terminal, a medical service providing server, or the like through the network unit 21.

Meanwhile, according to another embodiment of the present disclosure, operations performed by a processor may be implemented as software (e.g., program) including one or more instructions stored in a machine-readable storage medium.

For example, a processor of a machine may invoke and execute at least one of the one or more instructions stored from the storage medium. Accordingly, the machine is enabled to operate to perform at least one function according to the at least one invoked instruction. The one or more instructions may include code generated by a compiler or code executable by an interpreter. The machine-readable storage medium may be provided in the form of a non-transitory storage medium. Here, 'non-transitory' only means that the storage medium is a tangible device and does not contain a signal (for example, electromagnetic waves). This term does not distinguish a case where data is stored in the storage medium semi-permanently and a case where the data is stored in the storage medium temporarily.

Meanwhile, according to another embodiment of the present disclosure, each operation performed by a processor may be included and provided in a computer program product.

The computer program products are products that can be traded between sellers and buyers. The computer program product may be distributed in a form of machine-readable storage medium (for example, a compact disc read-only memory (CD-ROM)), or distributed through an application store (for example, Play Store™) or directly or online between two user devices (for example, download or upload). In the case of online distribution, at least a part of the computer program product may be at least temporarily stored or temporarily generated in the machine-readable storage medium such as a server of a manufacturer, a server of an application store, or a memory of a relay server.

Furthermore, in the specification, the term "unit" or "-or/er" may be a hardware component such as a processor or circuit and/or a software component that is executed by a hardware component such as a processor.

The scope of the present disclosure is defined by the appended claims rather than the detailed description, and all changes or modifications within the scope of the appended claims and their equivalents will be construed as being included in the scope of the present disclosure.

## Claims

1. A cognitive impairment determination method in which each operation is performed by a processor, comprising:

   determining N subjects who are subject to cognitive impairment determination and obtaining personal data of the subjects;
   obtaining clinical outcome data for the subjects;
   obtaining gait data for the subjects, based on gait measurement values measured by using inertia measurement units worn on centers of body mass of the subjects; and
   developing a cognitive impairment determination model including a plurality of predictors, based on obtained datasets,
   wherein the cognitive impairment determination model includes gait speeds and step time variability included in the gait data as some of the plurality of predictors.

2. The cognitive impairment determination method of claim 1, wherein the obtaining of the personal data comprises obtaining the personal data by receiving ages, sexes, educations, heights, weights, presence of lower limb arthritis, and mini mental state examination results of the subjects.

3. The cognitive impairment determination method of claim 1, wherein the obtaining of the clinical outcome data comprises, based on a result of diagnosing cognitive impairment including mild cognitive impairment and dementia, according to pre-set diagnostic criteria, receiving results of classifying subjects diagnosed with cognitive impairment into a first group and classifying subjects not diagnosed with cognitive impairment into a second group.

4. The cognitive impairment determination method of claim 1, wherein the obtaining of the gait data comprises:

   obtaining signals measured when the subjects walk by using the inertia measurement units worn on the centers of body mass of the subjects; and
   obtaining cadences, step times, gait speeds, step lengths, and step time variability, based on the obtained signals.

5. The cognitive impairment determination method of claim 1, wherein the developing of the cognitive impairment determination model comprises:

   dividing the datasets into development datasets and validation datasets;
   developing the cognitive impairment determination model including the gait speeds and the step time variability as some of the plurality of predictors, by using binary logistic regression analysis using a portion of the gait data and a portion of the personal data as independent variables, for the development datasets;
   developing a comparative model including mini mental state examination scores and ages as predictors, by using binary logistic regression analysis using a portion of the personal data as independent variables, for the development datasets; and
   generating receiver operating characteristics (ROC) curves of the developed cognitive impairment determination model and the developed comparative model, for the development datasets and the validation datasets, and evaluating determination performance of the cognitive impairment determination model by comparing areas under the curves (AUC).

6. The cognitive impairment determination method of claim 1, further comprising determining whether a determination target has cognitive impairment by using the developed cognitive impairment determination model.

7. The cognitive impairment determination method of claim 6, wherein the determining of whether the determination target has cognitive impairment comprises:

obtaining educations of the determination target;
receiving a tri-axial acceleration signal and a tri-axial angular velocity signal measured by the inertia measurement units worn on a center of body mass of the determination target and obtaining a gait speed and step time variability, based on the tri-axial acceleration signal and the tri-axial angular velocity signal; and
determining whether the determination target has cognitive impairment by substituting obtained values into the cognitive impairment determination model.

8. A computer program product comprising one or more computer-readable recording media storing a program for performing a cognitive impairment determination method comprising:

determining N subjects who are subject to cognitive impairment determination and obtaining personal data of the subjects;
obtaining clinical outcome data for the subjects;
obtaining gait data for the subjects, based on gait measurement values measured by using inertia measurement units worn on centers of body mass of the subjects;
developing a cognitive impairment determination model including a plurality of predictors, based on obtained datasets; and
determining whether a determination target has cognitive impairment by using the developed cognitive impairment determination model,
wherein the cognitive impairment determination model includes gait speeds and step time variability included in the gait data as some of the plurality of predictors.

9. A cognitive impairment determination device comprising:

an input and output unit configured to receive personal data and clinical outcome data of subjects and a determination target, and output determined cognitive impairment of the determination target; a network unit configured to receive gait measurement values measured by using inertia measurement units worn on centers of body mass of the subjects and the determination target;
a processor configured to control the input and output unit to obtain the personal data and clinical outcome data of the subjects and the determination target, who are targets of cognitive impairment determination, obtain gait data based on the received gait measurement values, develop a cognitive impairment determination model including a plurality of predictors based on obtained datasets, and determine whether the determination target input through the developed cognitive impairment determination model has cognitive impairment; and
a storage unit storing the obtained personal data, clinical outcome data, and gait data,
wherein the cognitive impairment determination model includes gait speeds and step time variability included in the gait data as some of the plurality of predictors.

# FIG. 1

10

BACK

NETWORK

30

USER TERMINAL

COGNITIVE IMPAIRMENT
DETERMINATION DEVICE —20

# FIG. 2

| | |
|---|---|
| DETERMINE N SUBJECTS | S110 |

↓

| | |
|---|---|
| OBTAIN PERSONAL DATA OF SUBJECTS | S120 |

↓

| | |
|---|---|
| OBTAIN CLINICAL OUTCOME DATA OF SUBJECTS | S130 |

↓

| | |
|---|---|
| OBTAIN GAIT DATA OF SUBJECTS | S140 |

↓

| | |
|---|---|
| DEVELOP COGNITIVE IMPAIRMENT DETERMINATION MODEL | S150 |

# FIG. 3

S140

| WEAR, BY SUBJECT, INERTIA MEASUREMENT UNIT AND PERFORM GAIT EVALUATION | ~S141 |

| RECEIVE SIGNALS MEASURED THROUGH INERTIA MEASUREMENT UNIT | ~S142 |

| GENERATE GAIT DATA INCLUDING CADENCE, STEP TIME, GAIT SPEED, STEP LENGTH, STEP TIME VARIABILITY, AND STEP TIME ASYMMETRY, BASED ON RECEIVED SIGNALS | ~S143 |

# FIG. 4

S150

DIVIDE DATASETS INTO DEVELOPMENT DATASETS AND
VALIDATION DATASETS —S151

DEVELOP COGNITIVE IMPAIRMENT DETERMINATION MODEL
INCLUDING GAIT FEATURE VALUES AS PREDICTORS
THROUGH LOGISTIC REGRESSION ANALYSIS,
FOR DEVELOPMENT DATASETS —S152

DEVELOP COMPARATIVE MODEL INCLUDING MMSE SCORES
AS PREDICTORS THROUGH LOGISTIC REGRESSION ANALYSIS,
FOR DEVELOPMENT DATASET —S153

EVALUATE PERFORMANCE OF DEVELOPED COGNITIVE
IMPAIRMENT DETERMINATION MODEL BY USING
COMPARATIVE MODEL AND VALIDATION DATASETS —S154

# FIG. 5

| | All | | | Development dataset | | | Validation dataset | | |
|---|---|---|---|---|---|---|---|---|---|
| | CN (n = 494) | CD (n = 101) | p* | CN (n = 395) | CD (n = 81) | p* | CN (n = 99) | CD (n = 20) | p* |
| Age (years) | 72.7 (5.1) | 75.7 (5.7) | <0.001 | 72.6 (5.1) | 75.6 (6.0) | <0.001 | 72.9 (5.0) | 76.5 (4.6) | 0.004 |
| Sex (women) | 258 (52.2) | 55 (54.5) | 0.683 | 201 (50.9) | 43 (53.1) | 0.718 | 57 (57.6) | 12 (60.0) | 0.841 |
| Education (years) | 13.4 (3.8) | 11.5 (4.8) | <0.001 | 13.4 (3.8) | 11.2 (4.9) | <0.001 | 13.1 (4.0) | 12.4 (4.2) | 0.465 |
| Height (cm) | 160.9 (8.1) | 158.5 (8.5) | 0.008 | 161.1 (8.2) | 158.2 (8.6) | 0.005 | 160.2 (8.0) | 159.6 (8.4) | 0.760 |
| Weight (kg) | 62.0 (8.8) | 60.3 (9.1) | 0.080 | 62.3 (8.9) | 61.0 (8.9) | 0.213 | 60.9 (8.1) | 57.8 (9.6) | 0.137 |
| Lower limb arthritis (yes) | 144 (29.1) | 34 (33.7) | 0.367 | 113 (28.6) | 28 (34.6) | 0.285 | 31 (31.3) | 6 (30.0) | 0.908 |
| MMSE (points) | 28.0 (1.7) | 25.4 (3.6) | <0.001 | 28.1 (1.7) | 25.2 (3.9) | <0.001 | 27.8 (1.9) | 26.0 (2.5) | <0.001 |
| Gait features | | | | | | | | | |
|   Cadence (steps/min) | 114.7 (9.1) | 111.0 (10.0) | <0.001 | 114.4 (9.1) | 110.9 (10.1) | 0.002 | 115.6 (9.0) | 111.6 (9.9) | 0.075 |
|   Step time (ms) | 526.5 (42.1) | 545.0 (50.8) | 0.001 | 527.6 (42.2) | 545.8 (51.9) | 0.004 | 522.0 (41.3) | 541.7 (47.5) | 0.061 |
|   Step time variability (%) | 3.2 (0.8) | 4.0 (1.2) | <0.001 | 3.2 (0.8) | 3.9 (1.3) | <0.001 | 3.2 (0.9) | 4.5 (1.1) | <0.001 |
|   Step time asymmetry (ms) | 16.3 (15.6) | 20.7 (17.2) | 0.011 | 16.2 (15.4) | 22.0 (18.3) | 0.009 | 16.8 (16.2) | 15.6 (10.4) | 0.757 |
|   Gait speed (cm/s) | 115.2 (16.1) | 101.2 (15.7) | <0.001 | 115.2 (16.3) | 101.5 (15.9) | <0.001 | 115.3 (14.9) | 100.1 (14.8) | <0.001 |
|   Step length (cm) | 60.2 (5.9) | 54.6 (6.1) | <0.001 | 60.3 (6.1) | 54.8 (6.2) | <0.001 | 59.7 (5.1) | 53.7 (5.6) | <0.001 |

EP 4 483 787 A1

FIG. 6

|  | B (SE) | p | OR | 95%CI |
|---|---|---|---|---|
| Gait-based model |  |  |  |  |
| Constant | 2.286 (1.274) | 0.073 | 9.834 | – |
| Gait speed (cm/s) | – 0.040 (0.009) | <0.001 | 0.961 | 0.944 – 0.978 |
| Gait variability (%) | 0.535 (0.145) | <0.001 | 1.708 | 1.287 – 2.268 |
| Education (years) | – 0.115 (0.030) | <0.001 | 0.891 | 0.840 – 0.945 |
| MMSE-based model |  |  |  |  |
| Constant | 5.977 (2.678) | 0.026 | 394.192 | – |
| MMSE (score) | – 0.429 (0.060) | <0.001 | 0.651 | 0.579 – 0.733 |
| Age (years) | 0.055 (0.026) | 0.033 | 1.056 | 1.004 – 1.110 |

# FIG. 7A

gait-based model and MMSE score (p=0.381)
MMSE-based model and MMSE score (p=0.800)
gait-based model and MMSE-based model (p=0.542)

—— gait-based model
AUC = 0.788, 95%CI = 0.748-0.823, p < 0.001

•••• MMSE-based model
AUC = 0.762, 95%CI = 0.721-0.799, p < 0.001

—— MMSE score
AUC = 0.750, 95%CI = 0.709-0.788, p < 0.001

## FIG. 7B

gait-based model and MMSE score (p=0.233)
MMSE-based model and MMSE score (p=0.772)
gait-based model and MMSE-based model (p=0.339)

| | |
|---|---|
| —— gait-based model | AUC = 0.811, 95%CI = 0.729-0.877, p < 0.001 |
| ••••• MMSE-based model | AUC = 0.741, 95%CI = 0.653-0.817, p < 0.001 |
| —— MMSE score | AUC = 0.716, 95%CI = 0.627-0.795, p < 0.001 |

# FIG. 7C

gait-based model (p=0.666)

| | Development dafaset<br>AUC = 0.788, 95%CI = 0.748–0.823, p < 0.001 |
| --- | --- |

| ••••• | Vaildation dataset<br>AUC = 0.811, 95%CI = 0.729–0.877, p < 0.001 |
| --- | --- |

FIG. 8

|  | MMSE-based model | MMSE |
|---|---|---|
| All | 0.417 (<0.001) | −0.374 (<0.001) |
| CD group | 0.278 (0.005) | −0.255 (0.010) |
| CN group | 0.319 (<0.001) | −0.260 (<0.001) |

# FIG. 9

20

NETWORK UNIT ~ 21

STORAGE UNIT ~ 22

PROCESSOR ~ 23

INPUT AND
OUTPUT UNIT ~ 24

# FIG. 10

20

NETWORK UNIT ~21

PERSONAL DATA STORAGE UNIT ~221

GAIT DATA STORAGE AND GENERATION UNIT ~231

232
COGNITIVE IMPAIRMENT DETERMINATION UNIT

222
DETERMINATION RESULT STORAGE UNIT

24— INPUT AND OUTPUT UNIT

# FIG. 11

RECEIVE PERSONAL DATA OF DETERMINATION TARGET — S201

RECEIVE AND STORE SIGNALS FROM INERTIA MEASUREMENT UNIT — S202

GENERATE GAIT DATA FROM STORED SIGNALS — S203

DETERMINE WHETHER DETERMINATION TARGET HAS COGNITIVE IMPAIRMENT BY SUBSTITUTING PERSONAL DATA AND GAIT DATA INTO COGNITIVE IMPAIRMENT DETERMINATION MODEL — S204

PROVIDE DETERMINATION RESULT TO USER — S205

<div align="center">**INTERNATIONAL SEARCH REPORT**</div>

| International application No. |
|---|
| **PCT/KR2023/002087** |

**A. CLASSIFICATION OF SUBJECT MATTER**

**A61B 5/00**(2006.01)i; **A61B 5/11**(2006.01)i

According to International Patent Classification (IPC) or to both national classification and IPC

**B. FIELDS SEARCHED**

Minimum documentation searched (classification system followed by classification symbols)

A61B 5/00(2006.01); A61B 10/00(2006.01); A61B 5/0476(2006.01); A61B 5/11(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 신상 (personal details), 임상 (clinical), 인지 장애 (cognitive disorder), 보행 (walk), 예측 (prediction), 모델 (model)

**C. DOCUMENTS CONSIDERED TO BE RELEVANT**

| Category* | Citation of document, with indication, where appropriate, of the relevant passages | Relevant to claim No. |
|---|---|---|
| X | KR 10-2323818 B1 (JEIOS INC.) 09 November 2021 (2021-11-09)<br>See paragraphs [0114]-[0156]; claims 1 and 4; and figure 7. | 1-4,6-9 |
| A | | 5 |
| A | JP 2019-523027 A (NEUROVISION IMAGING LLC) 22 August 2019 (2019-08-22)<br>See entire document. | 1-9 |
| A | US 2019-0142338 A1 (LVIS CORPORATION) 16 May 2019 (2019-05-16)<br>See entire document. | 1-9 |
| A | KR 10-2102930 B1 (VITAL6THLAB. CO., LTD.) 22 April 2020 (2020-04-22)<br>See entire document. | 1-9 |
| A | US 2020-0008735 A1 (PANASONIC INTELLECTUAL PROPERTY MANAGEMENT CO., LTD.) 09 January 2020 (2020-01-09)<br>See entire document. | 1-9 |

☐ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **22 May 2023** | **22 May 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

| | | | | |
|---|---|---|---|---|
| **INTERNATIONAL SEARCH REPORT**<br>**Information on patent family members** | | International application No.<br>**PCT/KR2023/002087** | | |

| Patent document<br>cited in search report | | Publication date<br>(day/month/year) | Patent family member(s) | | Publication date<br>(day/month/year) |
|---|---|---|---|---|---|
| KR 10-2323818 | B1 | 09 November 2021 | KR 10-2021-0084846 | A | 08 July 2021 |
| JP 2019-523027 | A | 22 August 2019 | AU 2017-262666 | A1 | 22 November 2018 |
| | | | AU 2017-262666 | A2 | 06 December 2018 |
| | | | CA 3023659 | A1 | 16 November 2017 |
| | | | EP 3454727 | A1 | 20 March 2019 |
| | | | US 2017-0319063 | A1 | 09 November 2017 |
| | | | WO 2017-196785 | A1 | 16 November 2017 |
| US 2019-0142338 | A1 | 16 May 2019 | AU 2018-365070 | A1 | 28 May 2020 |
| | | | CA 3082082 | A1 | 16 May 2019 |
| | | | CN 111801046 | A | 20 October 2020 |
| | | | EP 3706617 | A1 | 16 September 2020 |
| | | | EP 3706617 | A4 | 18 August 2021 |
| | | | IL 274548 | A | 30 June 2020 |
| | | | IL 274548 | D0 | 30 June 2020 |
| | | | JP 2021-502165 | A | 28 January 2021 |
| | | | KR 10-2020-0086316 | A | 16 July 2020 |
| | | | SG 11202004256 | A | 29 June 2020 |
| | | | US 2021-0169411 | A1 | 10 June 2021 |
| | | | WO 2019-094836 | A1 | 16 May 2019 |
| KR 10-2102930 | B1 | 22 April 2020 | KR 10-2019-0123187 | A | 31 October 2019 |
| US 2020-0008735 | A1 | 09 January 2020 | CN 109862831 | A | 07 June 2019 |
| | | | CN 109862831 | B | 04 March 2022 |
| | | | JP 6771162 | B2 | 21 October 2020 |
| | | | SG 11201902960 | A | 30 May 2019 |
| | | | TW 201815349 | A | 01 May 2018 |
| | | | TW I655932 | B | 11 April 2019 |
| | | | WO 2018-066422 | A1 | 12 April 2018 |

Form PCT/ISA/210 (patent family annex) (July 2022)

**REFERENCES CITED IN THE DESCRIPTION**

**Patent documents cited in the description**

- WO 1020210194313 A

- WO 1020160124602 A

**Non-patent literature cited in the description**

- **J. W. HAN** ; **T. H. KIM** ; **K. P. KWAK** ; **K. KIM** ; **B. J. KIM** ; **S. G. KIM** ; **J. L. KIM** ; **T. H. KIM** ; **S. W. MOON** ; **J. Y. PARK**. Overview of the Korean longitudinal study on cognitive aging and dementia. *Psychiatry investigation*, 2018, vol. 15 (8), 767

- **MCKEITH IG** ; **DICKSON D** ; **LOWE J et al.** Diagnosis and management of dementia with Lewy bodies, third report of the DLB consortium. *Neurology*, 2005, vol. 65, 1863-1872

- **BLUMEN HM** ; **BROWN LL** ; **HABECK C et al.** Gray matter volume covariance patterns associated with gait speed in older adults: a multi-cohort MRI study. *Brain imaging and behavior*, 2019, vol. 13, 446-460

- **TIAN Q** ; **CHASTAN N** ; **BAIR W-N** ; **RESNICK SM** ; **FERRUCCI L** ; **STUDENSKI SA**. The brain map of gait variability in aging, cognitive impairment, and dementia-a systematic review. *Neuroscience & Biobehavioral Reviews*, 2017, vol. 74, 149-162

- **HANLEY JA** ; **MCNEIL BJ**. A method of comparing the areas under receiver operating characteristic curves derived from the same cases. *Radiology*, 1983, vol. 148, 839-843